# EUROPÄISCHE PATENTANMELDUNG

(11) **EP 1 400 553 A2**
(43) Veröffentlichungstag der Anmeldung: **24.03.2004**
(21) Anmeldenummer: 03025060.9
(22) Anmeldetag: 14.02.2001
(51) Int. Cl.: C08G 75/14, C07C 319/22, C07C 321/14, C08J 3/24

(54) **Polyalkylenpolysulfide**

(30) Priorität: 15.02.2000 DE 10006546; 27.03.2000 DE 10015176; 19.07.2000 DE 10035077
(62) Teilanmeldung aus: 01903769.6
(71) Anmelder: BASF AKTIENGESELLSCHAFT, 67056 Ludwigshafen (DE)
(72) Erfinder: Faderl, Jürgen Dr., 68549 Ilvesheim (DE); Sterzel, Hans-Josef Dr., 67125 Dannstadt-Schauernheim (DE)
(74) Vertreter: Isenbruck, Günter, Dr.

(57) **Zusammenfassung**

Polyalkylenpolysulfide, enthaltend Ketten der allgemeinen Formel I 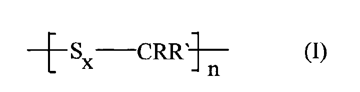 worin
- R und R': gleich oder verschieden sind und Wasserstoff oder eine Alkylgruppe mit 1 bis 4 C-Atomen oder COR", worin
- R" bedeuten,: Wasserstoff oder eine Alkylgruppe mit 1 bis 4 C-Atomen ist,
- x: eine Zahl von im Mittel 2 bis 20 ist und
- n: eine Zahl > 10 ist.

Offenbart ist ferner ein Verfahren zur Herstellung von Polyalkylenpolysulfiden durch Umsetzung von mindestens einer Carbonylverbindung der allgemeinen Formel II 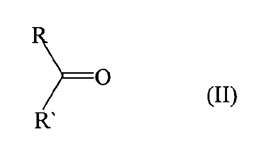 worin
- R und R': die vorstehend genannten Bedeutungen haben, mit Schwefel und Schwefelwasserstoff in Gegenwart eines basischen Katalysators.

## Beschreibung

Die Erfindung betrifft Polyalkylenpolysulfide, Verfahren zu ihrer Herstellung, ihre Verwendung, diese enthaltende Kautschuk-Zusammensetzungen, deren Verwendung, Vulkanisationsmittel, enthaltend die Polyalkylenpolysulfide, bestimmte, die Polyalkylenpolysulfide enthaltende Formmassen, daraus hergestellte Formteile, Verfahren zur Herstellung der Formteile sowie die Verwendung dieser Formmassen.

Polysulfidkautschuke finden hauptsächlich als Dichtmaterialien Anwendung. Feste Typen können dabei zu Dichtringen verarbeitet werden, während flüssige Typen beispielsweise als Fugendichtmassen dienen. Polysulfidkautschuke werden überlicherweise, beispielsweise wie in DE-A 675 401 beschrieben, durch Salzeliminierungsreaktionen aus Alkali-, Ammonium- oder Erdalkalipolysulfiden und Alkylendichloriden dargestellt. Nachteilig ist der enorme Salzanfall bei diesem Herstellungsverfahren.

In US 2,206,641 wird ein Verfahren zur Herstellung von Methylenpolysulfid beschrieben, bei dem Methylenchlorid mit Na₂S₂ in wässriger Lösung umgesetzt wird.

Lagert man unvulkanisierte Kautschukmischungen, denen als Vulkanisiermittel Sg-Schwefel in einer Menge oberhalb der Löslichkeitsgrenze zugesetzt wurde, über einen längeren Zeitraum, so kristallisiert an der Oberfläche der Kautschukmischungen der bei normalen Lagertemperaturen in Kautschuk schlecht lösliche S₈-Schwefel aus. Zur Vermeidung dieses als "Ausblühen" bezeichneten Effektes wird in der Gummiindustrie anstelle von S₈-Schwefel meist polymerer µ-Schwefel als Vulkanisiermittel verwendet. µ-Schwefel ist jedoch nicht langzeitstabil und zersetzt sich beim Lagern bereits bei Raumtemperatur zu S₈-Schwefel.

Um diesen Lagerproblemen zu begegnen, wurden mehrere Versuche unternommen, Vulkanisiermittel auf der Basis von Schwefel-Copolymerisaten zu entwickeln. Dabei handelt es sich um Copolymerisate mit Olefinen oder Olefinmischungen, insbesondere mit Dicyclopentadien und Styrol. Derartige Vulkanisiermittel, welche durch Reaktion von Schwefel mit einem Olefin bei 140°C bis 160°C in Gegenwart eines basischen Katalysators erhalten werden, sind in US 4,739,036, US 4,740,559 und US 2,989,513 offenbart.

Die gemäß der vorgenannten Schriften erhaltenen Produkte liegen in ihren Eigenschaften zwischen polymerem Schwefel und S₈-Schwefel und stellen daher gegenüber polymerem Schwefel keine Verbesserung dar. Insbesondere enthalten sie einen hohen Anteil an löslichem S₈-Schwefel, der zu einem schlechteren Ausblühverhalten als bei µ-Schwefel führt.

Aufgabe der Erfindung ist es Zusammensetzungen bereitzustellen, die als Abdichtmaterial, Beschichtungsstoff, Abdruckmasse und Formmasse zur Herstellung von Gummiartikeln und Folien sowie für weitere Anwendungszwecke geeignet sind.

Gelöst wird die Aufgabe durch Polyalkylenpolysulfidkautschuk-Zusammensetzungen, enthaltend 10 - 95 Gew.-% Polyalkylenpolysulfide, enthaltend Ketten der allgemeinen Formel I 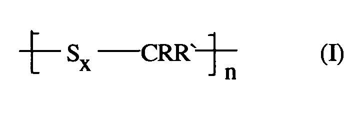 worin
- R und R': gleich oder verschieden sind und Wasserstoff oder eine Alkylgruppe mit 1 bis 4 C-Atomen oder COR", worin
- R" bedeuten,: Wasserstoff oder eine Alkylgruppe mit 1 bis 4 C-Atomen ist,
- x: eine Zahl von im Mittel 2 bis 20 ist und
- n: eine Zahl > 10 ist,
- als Komponente A, sowie 0,1 - 20 Gew.-%: eines Vernetzers als Komponente B,
- 0,1 - 90 Gew.-%: Füllstoffe und/oder Pigmente als Komponente C,
- 0 - 50 Gew.-%: Weichmacher als Komponente D und
- 0 - 20 Gew.-%: übliche Additive, wie Haftvermittler, Thixotropiermittel, Beschleuniger usw. als Komponente E.

Derartige Polyalkylenpolysulfidkautschuk-Zusammensetzungen können als Abdichtmaterial, Beschichtungsstoff, Abdruckmasse und Formmasse zur Herstellung von Gummiartikeln und Folien verwendet werden.

Die Polyalkylenpolysulfide A enthalten Ketten der allgemeinen Formel I und bestehen vorzugsweise im wesentlichen aus Ketten der allgemeinen Formel I. Im wesentlichen aus Ketten der allgemeinen Formel I bestehende Polyalkylenpolysulfide sind solche, die ganz überwiegend, beispielsweise zu mindestens 90%, vorzugsweise ausschließlich, Wiederholungseinheiten der allgemeinen Formel Ia 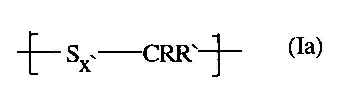 wobei x eine ganze Zahl ist, deren Wert von Wiederholungseinheit zu Wiederholungseinheit variieren kann und deren Mittelwert x ist, aufweisen. Die Polyalkylenpolysulfide weisen daneben Endgruppen auf. Als Endgruppen in Frage kommen u.a. SH, OH und H.

R und R' sind gleich oder verschieden und bedeuten Wasserstoff, Alkylgruppen mit 1 - 4 C-Atomen, wie Methyl, Ethyl, n- , iso-, sek- und tert-Butyl, oder COR", wobei R" für Wasserstoff oder eine der vorstehend genannten Alkylgruppen steht. x ist eine Zahl von im Mittel 2 bis 20, vorzugsweise 2 bis 5, besonders bevorzugt ca. 4, n ist eine Zahl > 10. Die Polyalkylenpolysulfide können niedermolekular oder hochmolekular sein. Niedermolekular sind beispielsweise Polyalkylenpolysulfide mit n = 10 - 50, hochmolekular sind beispielsweise solche mit n > 50. Vorzugsweise ist die Verteilung von x eng, d.h. in einem Polyalkylen-polysulfid, in dem x eine Zahl von beispielsweise im Mittel 4 ist, beträgt x' in
> 80% der Wiedeholungseinheiten der allgemeinen Formel Ia 4 und in jeweils
< 20% der Wiederholungseinheiten Ia 3 oder 5.

Die Reste R und R' können immer gleich sein oder können innerhalb der Kette variieren. Vorzugsweise sind die Reste R und R' immer gleich.

Die Polyalkylenpolysulfide A können durch Copolymerisation von Carbonylverbindungen mit elementarem Schwefel und Schwefelwasserstoff in Gegenwart eines basischen Katalysators hergestellt werden, nämlich durch Umsetzung von mindestens einer Carbonylverbindung der allgemeinen Formel II 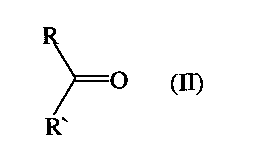 worin
- R und R': die vorstehend genannten Bedeutungen haben, mit Schwefel und Schwefelwasserstoff in Gegenwart eines basischen Katalysators gemäß der Gleichung: 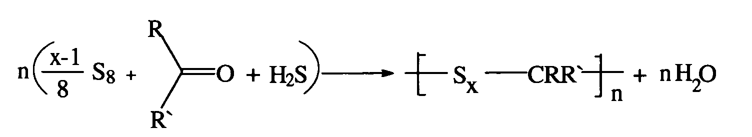
Geeignete Carbonylverbindungen sind Aldehyde R-CHO, worin vorzugsweise R=H, CH₃, C₂H₅, n- und iso-C₃H₇, n-, iso-, sek- und tert-C₄H₉ bedeutet. Geeignete Carbonylverbindungen sind ferner Ketone R-CO-R', worin R und R' gleich oder verschieden sein können und vorzugsweise CH₃, C₂H₅, n- und iso-C₃H₇ bedeuten. Geeignete Carbonylverbindungen sind ferner Glyoxale R-CO-CO-R" worin R und R" gleich oder verschieden sein können und vorzugsweise H, CH₃, C₂H₅, n- und iso-C₃H₇ bedeuten.
Besonders bevorzugte Carbonylverbindungen sind Formaledehyd, insbesondere als Formalin-Lösung, und Acetaldehyd.

Es können eine oder ein Gemisch aus mehreren verschiedenen Carbonyl-verbindungen der allegemeinen Formel II umgesetzt werden. Vorzugsweise wird genau eine Carbonylverbindung umgesetzt.

Der Wert von x kann durch Wahl des Molverhältnisses Carbonylverbindungen: elementarer Schwefel eingestellt werden. Das genannte Molverhältnis beträgt im allgemeinen von 1:1 bis 1:19, vorzugsweise von 1:2 bis 1:6 und besonders bevorzugt ca. 1:3 bezogen auf Schwefelatome. Beispielsweise werden mit einem Molverhältnis von ca. 1:3 Polyalkylenpolysulfide mit einem x von im Mittel ca. 4 gebildet. Die Stöchiometrie der Umsetzung ist derart, daß x-1 S-Atome aus dem elementaren (S₈)-Schwefel und ein S-Atom aus H₂S stammen.

Die Umsetzung wird in Gegenwart eines basischen Katalysators durchgeführt. Bevorzugte basische Katalysatoren sind solche mit einem sulfidischen Schwefelatom, wie Alkali-, Erdalkali- oder Ammoniumsulfide, -hydrogensulfide oder -polysulfide, besonders bevorzugt sind Na₂S, NaHS und (NH₄)HS. Ferner können Ammoniak, Amine und Hydroxyverbindungen wie NH₃, NBu₃ und NaOH eingesetzt werden, wobei bei Verwendung der letztgenannten, nicht sulfidischen Basen während der Reaktion mit H₂S sulfidische Verbindungen gebildet werden.

Das Verfahren wird vorzugsweise in wässrigem Medium bei einer Temperatur von im allgemeinen 117 bis 160°C, vorzugsweise 120 bis 140°C, besonders bevorzugt 122 bis 132°C, durchgeführt.

In einer Ausführungsform des Verfahrens wird in einem Autoklaven die Carbonylverbindung oder eine wässrige Verbindung der Carbonylverbindung mit elementarem Schwefel und dem basischen Katalysator vorgelegt und unter Schmelzen des Schwefels auf die oben angegebenen Temperaturen erhitzt. Anschließend wird Schwefelwasserstoff aufgepreßt, wobei mit einem Überdruck von beispielsweise 3 bar gearbeitet wird. Das Verfahren kann, muß jedoch nicht, mit einem Schwefelwasserstoff-Überdruck durchgeführt werden, vorzugsweise wird es so durchgeführt. Die Schwefelwasserstoffaufnahme stoppt üblicherweise von selbst bei Erreichen der stöchometrischen Menge, d. h. ein Mol Schwefelwasserstoff pro Mol Carbonylverbindung.

Wird die Carbonylverbindung in Form ihrer wässrigen Lösung eingesetzt, liegt neben der organischen Produktphase im allgemeinen noch eine wässrige Phase vor. Diese kann beispielsweise durch Abdekantieren oder in einem Phasenscheider von der organischen Phase abgetrennt werden. Die wässrige Phase kann auch, gegebenenfalls gemeinsam mit anderen flüchtigen Anteilen, durch Abdestillieren von der organischen Produktphase getrennt werden.

Das Verfahren kann diskontinuierlich oder kontinuierlich durchgeführt werden. Kontinuierlich wird es bevorzugt unter Verwendung einer Rührkesselkaskade durchgeführt, wobei die Abtrennung der wässrigen Phase durch Abdestillieren oder in einem Phasenscheider erfolgt.

Die Abtrennung der wässrigen Phase kann beim Einsatz der reinen Carbonylverbindung entfallen.

Das erhaltene Produkt kann durch Extraktion mit organischen, schwefellösenden Lösungsmitteln wie CS₂, CHCl₃ und CH₂Cl₂ von unumgesetztem Schwefel und Nebenprodukten, wie kleinen Kohlenstoff-Schwefel-Ringen, gereinigt werden.

Die Polyalkylenpolysulfide weisen eine Schmelzviskosität, extrapoliert auf die Schergeschwindigkeit 0s⁻¹, von 1 - 10⁴ Pa s, bevorzugt 5 - 1000 Pa s, auf.

Die Polyalkylenpolysulfide können, je nachdem, ob es sich um niedrigmolekulare oder hochmolekulare Massen handelt, ob diese unvulkanisiert oder vulkanisiert eingesetzt werden und ob sie weitere, verstärkend wirkende und die Erweichungstemperatur anhebende Füllstoffe enthalten, auf den verschiedensten Anwendungsgebieten eingesetzt werden.

So können die Polyalkylenpolysulfide zur Herstellung von Vulkanisationsmitteln, Abdichtmaterialien, Beschichtungsstoffen, Abdruckmassen, Klebstoffen, Kabel-isolierungen sowie von Formmassen zur Herstellung von Gummiartikeln und Folien verwendet werden.

Die Polyalkylenpolysulfide weisen unterschiedlich starke Klebrigkeiten auf. Sie können durch Einmischen von Füllstoffen in trockene, rieselfähige Pulver oder Granulate überführt werden. Die Polyalkylenpolysulfide können durch Zugabe von Vernetzern, wie ZnO, aktiviertem MnO₂, Perboraten oder Peroxiden vulkanisiert oder durch Reaktion mit Isocyanaten, Epoxiden und Doppelbindungen enthaltenden Verbindungen vernetzt werden. Sie verlieren dadurch ihre thermoplastischen Eigenschaften.

Niedrigmolekulare Polyalkylenpolysulfide können bei Raumtemperatur verarbeitet werden und lassen sich als Abdichtmaterial oder Korrosionsschutz auf Flächen wie Beton, Kunststein, Naturstein und Metalloberflächen durch Streichen, Rakeln oder Spritzen auftragen. Mischt man kurz vor der Verarbeitung Vulkanisationsmittel zu, so werden die flächig aufgetragenen Schichten vernetzt und damit formstabil. Höhermolekulare Polyalkylenpolysulfide lassen sich bei Temperaturen von beispielsweise 20 bis 120°C zu Profilen und Bändern extrudieren, wobei sie mit verstärkend wirkenden Füllstoffen abgemischt werden können. Derart ausgerüstet, lassen sie sich durch Breitschlitzdüsen zu Folien mit Dicken von beispielsweise 1 bis 20 mm extrudieren, die ebenfalls als Abdichtmaterialien eingesetzt werden können.

Die Polyalkylenpolysulfide sind gegen Chemikalien und Öle beständig und quellen nicht, wodurch sie, insbesondere für die Beschichtung von Reaktionsapparaten, Chemikalienbehältern, Ventilen und Rohrleitungen geeignet sind.
Die erfindungsgemäßen Polyalkylenpolysulfidkautschuk-Zusammensetzungen enthalten 10 - 95 Gew.-%, vorzugsweise 20 - 70 Gew.-% eines Polyalkylen-polysulfids als Komponente A.

Die erfindungsgemäßen Polyalkylenpolysulfidkautschuk-Zusammensetzungen enthalten 0,1 - 20 Gew.-%, vorzugsweise 1 - 15 Gew.-%, eines Vernetzers als Komponente B. Geeignete Vernetzer sind anorganische Vernetzer wie Bleidioxid, Mangandioxid, Kaliumpermangant, Chromate, Dichromate, Alkaliperborate, Calciumperoxid, Lithiumperoxid, Zinkperoxid und organische Vernetzer wie Hydroperoxide, beispielsweise Cumolhydroperoxid, Dioxime, Di- und Polyisothiocyanate.

Die erfindungsgemäßen Polyalkylensulfidkautschuk-Zusammensetzungen enthalten 0,1 - 90 Gew.-%, vorzugsweise 1 - 50 Gew.-% Füllstoffe und/oder Pigmente als Komponente C. Geeignete Füllstoffe sind beispielsweise feinteiliges Siliciumdioxid, Titandioxid, Talkum, Calciumcarbonat, Kaolin und Ruß. Pigmente sind beispielsweise Titandioxid, Eisenoxid und Ruß.

Die erfindungsgemäßen Polyalkylenpolysulfidkautschuk-Zusammensetzungen können ferner 0 - 50 Gew.-% Weichmacher als Komponente D enthalten. Weichmacher sind beispielsweise Phthalsäureester, Benzylbutylphthalat und Chlorparaffine.

Die erfindungsgemäßen Polyalkylenpolysulfidkautschuk-Zusammensetzungen können ferner 0 - 20 Gew.-% übliche Aditive wie Haftvermittler, Thixotro-piermittel, Beschleuniger, Verzögerer und Trocknungsmittel als Komponente E enthalten.

Die erfindungsgemäßen Polyalkylenpolysulfidkautschuk-Zusammensetzungen können sowohl als Einkomponenten-Systeme, als auch als Zweikomponenten-Systeme formuliert werden. Beispielsweise kann Komponente 1 die erfindungsgemäßen Polyalkylenpolysulfide und die Füllstoffe, gegebenenfalls neben Weichmachern und anderen Additiven enthalten, während Komponente 2 den Vernetzer, gegebenenfalls neben weiterem Weichmacher und weiteren Additiven, enthält, wobei Komponenten 1 und 2 unmittelbar vor der Anwendung vermischt werden.

Die Erfindung wird durch die nachfolgenden Beispiele näher erläutert.

### Beispiele

### Beispiel 1:

In einem 1,8 1-Autoklaven werden 576 g Schwefel, 247 g Formalinlösung (36,5 Gew.-% in Wasser) und 30 g Na₂S-Hydrat (35 Gew.-% Na₂S) vorgelegt und auf 125°C geheizt, wobei sich ein Druck von 5,2 bar aufbaut. Nach dem Schmelzen des Schwefels werden 102 g Schwefelwasserstoff aufgepreßt, wobei der Druck 8,0 bar nicht überschreitet. Nach Beendigung der Schwefelwasserstoff-Aufnahme wird noch eine Stunde lang gerührt. Der Autoklav wird bei ca. 100°C entleert und nach Erkalten der Reaktionsmischung die wässrige Phase entfernt. Es werden
720 g eines festen, gelblichen Polysulfids erhalten.

### Beispiel 2:

In einem 1 ,8 1-Autoklaven werden 481 g Schwefel, 411 g Formalinlösung (36,5 Gew.-% in Wasser) und 7 g NaHS vorgelegt und auf 125°C geheizt, wobei sich ein Druck von 4,7 bar aufbaut. Nach dem Schmelzen des Schwefels werden 147 g Schwefelwasserstoff aufgepreßt, wobei der Druck 8,0 bar nicht überschreitet. Nach Beendigung der Schwefelwassersoff-Aufnahme wird noch eine Stunde weitergerührt. Anschließend werden bei 100°C alle flüchtigen Komponenten abdestilliert. Nach Erkaltenlassen werden 625 g eines klebrigen, plastisch verformbaren, gelben Polysulfids erhalten.

### Beispiel 3:

In einem 1,4 1-Autoklaven werden 337 g Schwefel, 155 g Acetaldehyd, 231 g Wasser und 8,9 g NH₄HS (50 Gew.-% in H₂O) vorgelegt und auf 125°C geheizt, wobei sich ein Druck von 6,1 bar aufbaut. Nach dem Schmelzen des Schwefels werden 112 g Schwefelwasserstoff aufgepreßt, wobei der Druck 9,1 bar nicht überschreitet. Nach Beendigung der Schwefelwasserstoff-Aufnahme wird noch eine Stunde lang gerührt. Der Autoklav wird bei ca. 90°C entleert und nach Erkalten der Reaktionsmischung die wässrige Phase entfernt. Es werden 656 g eines festen, braunen Polysulfids erhalten.

### Beispiel 4:

In einem 1,8 1-Autoklaven werden 512 g Schwefel, 232 g Aceton, 300 g Wasser und 2,9 g Na₂S-Hydrat (35 Gew.-% Natriumsulfid) vorgelegt und auf 125°C geheizt, wobei sich ein Druck von 4,0 bar aufbaut. Nach dem Schmelzen des Schwefels werden 138 g Schwefelwasserstoff aufgepreßt, wobei der Druck 8,2 bar nicht überschreitet. Nach Beendigung der Schwefelwasserstoff-Aufnahme wird noch eine Stunde weitergerührt und der Autoklave bei 100°C entleert. Nach Erkaltenlassen und Entfernen der wässrigen Phase werden 768 g eines zähflüssigen, gelbbraunen Polysulfids erhalten.

### Beispiel 5:

In einem 1,4 1-Autoklaven werden 337 g Schwefel, 255 g Isobutyraldehyd, 376 g Wasser und 21,3 g Na₂S-Hydrat (32 Gew.-% Natriumsulfid) vorgelegt und auf 125°C geheizt, wobei sich ein Druck von 4,0 bar aufbaut. Nach dem Schmelzen des Schwefels werden 120 g Schwefelwasserstoff aufgepreßt, wobei ein Druck von 7,3 bar nicht überschritten wird. Nach Beendigung der Schwefelwasserstoff-Aufnahme werden bei 100 bis 125°C alle flüchtigen Komponenten abdestilliert. Nach Entleeren des Autoklaven und Erkaltenlassen werden 591 g eines gelbbraunen, viskosen Polysulfids erhalten.

### Beispiel 6:

In einer Knetmaschine werden 4 Gewichtsteile des klebrigen Polysulfids aus Beispiel 2 mit 5 Gewichtsteilen Kieselsäure verarbeitet, wobei ein rieselfähiges Pulver erhalten wird.

### Beispiel 6a:

In einem Schaufeltrockner werden 4 Gewichtsteile einer Kieselsäure bei 80°C vorgelegt und dann 6 Gewichtsteile eines klebrigen Polysulfids aus Beispiel 2 zugegeben, wobei ein rieselfähiges Pulver erhalten wird.

### Beispiel 7:

In einem Innenmischer werden 100 Gewichtsteile Naturkautschuk (Nerub 340 P® , Firma Weber & Schaer), 1 Gewichtsteil Stearinsäure, 8 Gewichtsteile Zinkoxid, 1,75 Gewichtsteile Alterungsschutzmittel auf Basis aromatischer Amine (0,75 Gewichtsteile Vulkanox 4010 Na und 1 Gewichtsteil Vulkanox DDA der Firma Bayer) und 50 Gewichtsteile Ruß (N330) 4 Minuten lang gemischt. Anschließend werden bei 60°C auf einem Walzwerk 3,6 Gewichtsteile des Polysulfids aus Beispiel 2, 0,65 Gewichtsteile des Vulkanisationsbeschleunigers 0,65 Gewichtsteile des Vulkanisationsbeschleunigers Thiazolylsufenamid (Vulkacit CZ der Firma Bayer) und 2 Gewichtsteile Vulkanisationsverzögerer auf Basis von Sulfonamiden (Vulkalent E der Firma Bayer) 8 Minuten lang eingearbeitet. Aus der erhaltenen Kautschukmischung werden Prüfkörper hergestellt und bei 150°C vulkanisiert. An den so hergestellten Prüfkörpern werden folgende Meßwerte bestimmt:

**Tabelle 1**

| | |
|---|---|
| Mooney-Scorch-Test nach DIN 53523 Teil 4 | |
| TS 5 bei 100°C: | > 60 Min |
| TS 5 bei 120°:C | 18 Min 28 Sek |
| Rheometermessungen bei 150°C nach DIN 53529 Teil 3 | |
| minimales Drehmoment: | 2,54 dNm |
| maximales Drehmoment: | 19,13 dNm |
| Δ Drehmoment (dNm): | 16,53 dNm |
| t₉₀ (Minuten): | 18,37 Min |
| t bei Reversion 99%: | > 60 Min |
| Shore A Härte nach DIN 53 505: | 68,0 +/- 0,6 |
| nach Alterung (48 Stunden bei 100°C): | 73,7 +/- 0,3 |
| Zugversuch S2 nach DIN 53 504 unmittelbar nach Vulkanisation | |
| Zugfestigkeit: | 27,6 +/- 0,3 MPa |
| Reißdehnung: | 496 +/- 10 % |
| Spannungswert 100%: | 3,4 MPa |
| Spannungswert 200%: | 8,9 +/- 0,1 MPa |
| Spannungswert 300%: | 15,3 +/- 0,2 MPa |
| Zugversuch S2 nach DIN 53504 nach Alterung (48 Stunden bei 100°C) | |
| Zugfestigkeit: | 19,6 +/-1,0 MPa |
| Reißdehnung: | 329 +/- 14% |
| Spannungswert 100%: | 4,7 +/- 0,1 MPa |
| Spannungswert 200%: | 11,5 +/- 0,3 MPa |
| Spannungswert 300%: | 18,1 +/- 0,4 MPa |
| Weiterreißwiderstand | |
| Streifenprobe nach DIN 53507: | 18,4 +/- 4,0 N/mm |
| nach Graves: | 37,1 +/- 3,0 N/mm |
| Flexometerprüfung | |
| Fließen: | 9,2 +/- 1,1% |
| Bleibende Verformung: | 11,3 +/- 0,2% |
| Temperaturerhöhung: | 25,0°C |

### Beispiel 7a:

In einem Innenmischer werden 100 Gewichtsteile Naturkautschuk (Nerub 340 P® , Firma Weber & Schaer), 1 Gewichtsteil Stearinsäure, 8 Gewichtsteile Zinkoxid, 1,75 Gewichtsteile Alterungsschutzmittel auf Basis aromatischer Amine (0,75 Gewichtsteile Vulkanox 4010 Na und 1 Gewichtsteil Vulkanox DDA der Firma Bayer) und 50 Gewichtsteile Ruß (N330) 4 Minuten lang gemischt. Anschließend werden bei 60°C auf einem Walzwerk 6,1 Gewichtsteile des Polysulfids aus Beispiel 6a, 0,65 Gewichtsteile des Vulkanisationsbeschleunigers 0,65 Gewichtsteile des Vulkanisationsbeschleunigers Thiazolylsufenamid (Vulkacit CZ der Firma Bayer) und 2 Gewichtsteile Vulkanisationsverzögerer auf Basis von Sulfonamiden (Vulkalent E der Firma Bayer) 8 Minuten lang eingearbeitet. Aus der erhaltenen Kautschukmischung werden Prüfkörper hergestellt und bei 150°C vulkanisiert. An den so hergestellten Prüfkörpern werden folgende Meßwerte bestimmt:

**Tabelle 2**

| | |
|---|---|
| Mooney-Scorch-Test nach DIN 53523 Teil 4 | |
| TS 5 bei 100°C: | > 60 Min |
| TS 5 bei 120°:C | 15,9 Min |
| Rheometermessungen bei 150°C nach DIN 53529 Teil 3 | |
| maximales Drehmoment: | 16,69 dNm |
| t₉₀ (Minuten): | 19,1 Min |
| Shore A Härte nach DIN 53 505: | 66,0 |
| nach Alterung (72 Stunden bei 100°C): | 71,0 |
| Zugversuch S2 nach DIN 53 504 unmittelbar nach Vulkanisation | |
| Zugfestigkeit: | 19,3 MPa |
| Reißdehnung: | 400,1 % |
| Zugversuch S2 nach DIN 53504 nach Alterung (72 Stunden bei 100°C) | |
| Zugfestigkeit: | 7,5 MPa |
| Reißdehnung: | 165,7% |
| Weiterreißwiderstand nach Graves: | 50,9 N/mm |

## Patentansprüche

1. Polyalkylenpolysulfidkautschuk-Zusammensetzung enthaltend
10 - 95 Gew.-% eines Polyalkylenpolysulfids enthaltend Ketten der allgemeinen Formel I 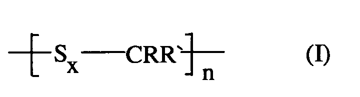 worin
R und R' gleich oder verschieden sind und Wasserstoff oder eine Alkylgruppe mit 1 bis 4 C-Atomen oder COR", worin
R" Wasserstoff oder eine Alkylgruppe mit 1 bis 4 C-Atomen ist, bedeuten,
x eine Zahl von im Mittel 2 bis 20 ist und
n eine Zahl > 10 ist, als Komponente A,
0,1 - 20 Gew.-% eines Vernetzers als Komponente B,
0,1 - 90 Gew.-% Füllstoffe und/oder Pigmente als Komponente C,
0 - 50 Gew.-% Weichmacher als Komponente D und
0 - 20 Gew.-% übliche Additive, wie Haftvermittler, Thixotropiermittel, Beschleuniger als Komponente E.

2. Polyalkylenpolysulfidkautschuk-Zusammensetzung nach Anspruch 1, **dadurch gekennzeichnet, daß** das Polyalkylenpolysulfid im wesentlichen aus Ketten der allgemeinen Formel (I) besteht und x eine Zahl von im Mittel 2 bis 5 ist.

3. Polyalkylenpolysulfidkautschuk-Zusammensetzung nach Anspruch 1 oder 2, **dadurch gekennzeichnet, dass** die Polyalkylenpolysulfide durch Umsetzung von mindestens einer Carbonyl-verbindung der allgemeinen Formel II 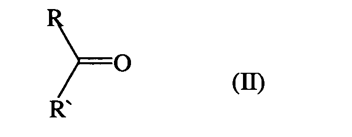 worin
R und R' die vorstehend genannten Bedeutungen haben,
mit Schwefel und Schwefelwasserstoff in Gegenwart eines basischen Katalysators hergestellt sind.

4. Polyalkylenpolysulfidkautschuk-Zusammensetzung nach Anspruch 3, **dadurch gekennzeichnet, daß** das Molverhältnis Carbonylverbindung: Schwefel, bezogen auf Schwefelatome, von 1:2 bis 1:6 beträgt.

5. Polyalkylenpolysulfidkautschuk-Zusammensetzung nach Anspruch 3 oder 4, **dadurch gekennzeichnet, daß** der basische Katalysator ausgewählt ist aus der Gruppe, bestehend aus Basen mit sulfidischen Schwefelatomen, Ammoniak, Aminen und Hydroxyverbindungen.

6. Verwendung einer Polyalkylenpolysulfidkautschuk-Zusammensetzung, wie sie in Anspruch 1 oder 2 definiert sind, als Abdichtmaterial, Beschichtungsstoff, Abdruckmasse und Formmasse zur Herstellung von Gummiartikeln und Folien.
